# FASCICULE DE BREVET EUROPEEN

(11) **EP 4 545 013 B1**
(45) Date de publication et mention de la délivrance du brevet: **18.03.2026**
(21) Numéro de dépôt: 24204779.3
(22) Date de dépôt: 04.10.2024
(51) Int. Cl.: A61B 8/12

(54) **SONDE DE DIAGNOSTIC BIMODALE**
BIMODALE DIAGNOSTISCHE SONDE
BIMODAL DIAGNOSTIC PROBE

(30) Priorité: 27.10.2023 FR 2311724
(43) Date de publication de la demande: 30.04.2025
(73) Titulaire: Commissariat à l'Energie Atomique et aux Energies Alternatives, 75015 Paris (FR); Université Grenoble Alpes, 38400 Saint-Martin-d'Hères (FR); Centre Hospitalier Universitaire Grenoble Alpes, 38043 Grenoble Cedex 9 (FR)
(72) Inventeur: RATEL, David, 38054 Grenoble cedex 09 (FR); POIZAT, Adrien, 38054 Grenoble cedex 09 (FR); MEYER, Mikael, 13008 Marseille (FR); GAY, Emmanuel, 38330 Montbonnot (FR)
(74) Mandataire: INNOV-GROUP

(56) Documents cités:
- US-A1- 2006 036 182
- US-A1- 2009 203 991

## Description

### Domaine technique de l'invention

La présente invention se rapporte à une sonde de diagnostic bimodale, destinée à être intégrée dans un système de diagnostic capable de générer des images à partir de données ultrasonores et de données optiques.

### Etat de la technique

Parmi les pathologies endocriniennes, les adénomes hypophysaires sont des tumeurs de bas grade à croissance lente représentant environ 10% des tumeurs cérébrales primaires. Elles peuvent entraîner la sécrétion de quantités anormalement élevées d'une ou plusieurs hormones (adénome corticotrope sécrétant l'ACTH pour la maladie de Cushing, adénome somatotrope sécrétant l'hormone de croissance ou GH pour l'acromégalie...). La maladie de Cushing (adénomes hypophysaires sécrétant de l'ACTH) est certainement l'une des plus délétères pour le patient.

L'exérèse complète de l'adénome corticotrope permet la guérison du patient. Cependant sa détection n'est pas toujours possible et cela malgré les progrès de l'imagerie par résonance magnétique (IRM).

L'imagerie par résonance magnétique est actuellement l'examen de choix pour localiser l'adénome. Cependant la visualisation à l'imagerie des adénomes sécrétants (dont ceux sécrétant de l'ACTH (hormone corticotrope)) est rendue délicate en raison de leur petite taille.

En outre, le traitement chirurgical demeure à ce jour le traitement de référence, celui-ci permettant d'obtenir la guérison quand l'exérèse est complète. La chirurgie de l'hypophyse est réalisée par une voie d'abord « endonasale » trans-sphénoïdale sous guidage endoscopique.

En cas d'adénome visible à l'IRM, le neurochirurgien connaît la localisation de l'adénome et va pouvoir atteindre l'adénome au moyen d'un endoscope permettant un contrôle visuel de l'exploration de la glande et de la résection de l'adénome. Toutefois, les images IRM et la visualisation endoscopique ne pouvant être superposées, l'exploration de la glande demeure difficile.

En cas d'adénome non visible à l'IRM, il est connu de proposer une hypophysectomie totale, conduisant à une insuffisance hypophysaire post-opératoire.

Cependant, il a récemment été mis en évidence un paramètre qui différencie le tissu adénomateux du tissu hypophysaire sain, ce paramètre étant la rigidité tissulaire (Meyer M and al., 2021). La glande hypophysaire et plus particulièrement l'hypophyse antérieure est en effet un tissu dont la matrice extracellulaire est riche en fibres de réticuline et de collagène IV.

L'étude de la caractérisation micromécanique des adénomes hypophysaires par comparaison avec l'hypophyse saine montre que la rigidité moyenne (exprimée en kilopascal ou kPa), mesurée par microscopie par force atomique (appelée AFM) dans les tissus adénomateux, diffère significativement de celle estimée dans la glande hypophysaire saine. En effet la rigidité moyenne des tissus adénomateux est comprise entre 0,1 et 0,2 kPa pour les adénomes (somatotrope et corticotrope) testés, alors que celle mesurée dans le parenchyme hypophysaire sain est d'environ 10 kPa (Meyer M and al., 2021).

La localisation peropératoire des lésions pourrait ainsi être évaluée par une approche basée sur la mesure de la rigidité au moyen d'une sonde non invasive (vis-à-vis de la glande) en utilisant la modalité ultrasonore couplée à la modalité optique.

Une sonde bimodale est connue du document US2009/203991 A1 et également à partir de US2006/036182A1.

Le but de l'invention est de disposer d'une sonde bimodale capable de mettre en œuvre à la fois une détection de l'adénome par mode ultrasonore et de permettre sa résection grâce au mode optique, ladite sonde :
- permettant la superposition de l'information ultrasonore (élastographique) à l'image optique habituellement utilisée par le neurochirurgien ;
- ayant un encombrement spatial adapté à l'introduction des instruments chirurgicaux couramment utilisés lors de ce type de chirurgie ;

De manière non limitative, la sonde de l'invention pourra notamment être employée dans les champs d'application suivants : exérèse des adénomes hypophysaires, les tumeurs cérébrales au sens large (méningiomes, gliomes, cavernomes, craniopharyngiomes, etc.), les tumeurs au sens large (foie, sein, poumon, côlon, vessie, prostate, thyroïde, ovaire).

### Exposé de l'invention

Ce but est atteint par une Sonde de diagnostic bimodale, employée pour examiner des tissus biologiques et destinée à être intégrée dans un système de diagnostic capable de générer des images à partir de données ultrasonores et de données optiques, ladite sonde comprenant :
- Un corps présentant une forme allongée suivant un axe longitudinal,
- Le corps comportant une partie distale destinée à venir au plus près des tissus à examiner,
- Au moins un dispositif ultrasonore comportant une matrice de transducteurs ultrasonores, ladite matrice de transducteurs ultrasonores étant commandée pour émettre des signaux ultrasonores en direction des tissus à examiner et pour convertir les signaux ultrasonores réfléchis en signaux électriques,
- Au moins un dispositif optique comportant une source lumineuse, ladite au moins une source lumineuse étant contrôlée pour émettre un faisceau lumineux en direction des tissus à examiner, le dispositif optique comprenant également au moins un capteur chargé de capter les signaux lumineux diffusés par les tissus,
- Le dispositif optique étant agencé pour émettre ledit faisceau lumineux au niveau de la partie distale de la sonde,
- La sonde comportant une partie montée mobile en coulissement dans ledit corps suivant une direction parallèle à l'axe longitudinal, ladite partie mobile comprenant une extrémité distale portant le dispositif ultrasonore,
- La sonde comportant des moyens de réglage de la position longitudinale de ladite partie mobile.

Ainsi, alors que le faisceau lumineux est émis via la partie distale de la sonde, la partie mobile qui porte le dispositif ultrasonore à son extrémité peut se déplacer en coulissement, permettant ainsi de jouer sur sa position longitudinale. Le faisceau lumineux est ainsi émis par la partie fixe de la sonde alors que les ondes ultrasonores peuvent être émises d'une position plus ou moins éloignée des tissus, selon la position prise par la partie mobile.

Selon une particularité, la sonde comporte un capteur de déplacement agencé pour détecter la position longitudinale de la partie mobile.

Selon une autre particularité, la sonde comporte un dispositif de blocage en coulissement de la partie mobile.

Selon une autre particularité, la sonde comporte au moins un capteur de pression mécanique situé à l'extrémité distale de la partie mobile.

Selon une autre particularité, le dispositif ultrasonore comporte une matrice de transducteurs ultra-sonores.

Selon une autre particularité, la sonde comporte un canal dit canal opérateur intégré audit corps de la sonde et s'étendant selon une direction parallèle à l'axe longitudinal.

L'invention est décrite dans la suite de revendications ci-jointe.

### Brève description des figures

D'autres caractéristiques et avantages vont apparaître dans la description détaillée qui suit faite en regard des dessins annexés dans lesquels :
- La figure 1 montre un exemple de réalisation de la sonde bimodale de l'invention ;
- Les figures 2A à 2D montrent plusieurs variantes de réalisation de la partie distale de la sonde bimodale de l'invention ;
- La figure 3 montre de manière schématique l'architecture matérielle et logicielle du système de diagnostic de l'invention intégrant ladite sonde bimodale ;

### Description détaillée d'au moins un mode de réalisation

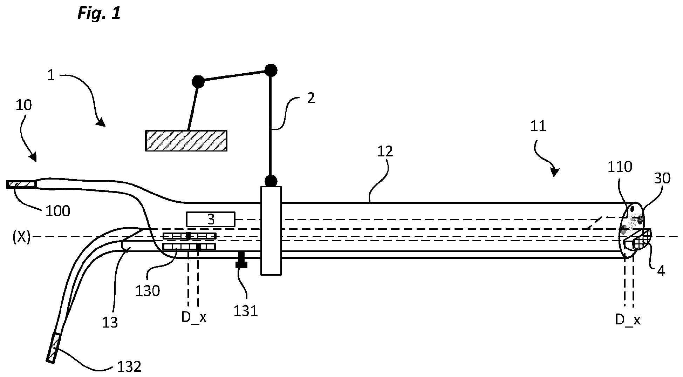

En référence à la figure 1, selon l'invention, la sonde 1 bimodale comporte un corps 12 de forme allongée suivant un axe longitudinal (X). La sonde 1 comporte une première partie dite proximale 10 et une deuxième partie dite distale 11 opposée.

La partie proximale 10 de la sonde comporte des moyens 100 de préhension et de manipulation de la sonde 1 par un opérateur (par exemple le chirurgien).

La sonde 1 de l'invention peut notamment être montée sur un bras articulé 2, qui de manière optionnelle pourra être piloté par l'unité de contrôle et de traitement (voir ci-après).

La partie distale 11 de la sonde 1 comporte des moyens de détection, destinés à être approchés des tissus à examiner.

Les moyens de détection de la sonde bimodale comportent un dispositif optique 3 et un dispositif ultrasonore 4.

La partie distale 11 de la sonde comporte avantageusement une face 110 dite avant orientée perpendiculairement à son axe longitudinal (X). Cette face avant 110 est destinée à venir en vis-à-vis des tissus à examiner.

Le dispositif optique 3 comporte au moins une source lumineuse. La source lumineuse peut comporter une ou plusieurs diodes électroluminescentes en position distale de la sonde ou à distance en couplage avec au moins une fibre optique pour acheminer la lumière à travers le corps 12 de la sonde (comme sur la figure 1).

Le faisceau lumineux 30 est délivré au niveau de la partie distale 11 de la sonde, à travers la face avant 110 de la sonde 1 et orientée de manière à être émis en direction des tissus à examiner.

Le dispositif optique 3 comporte également au moins une caméra optique chargée de capter les ondes lumineuses réfléchies par les tissus, après illumination. La caméra optique peut être située en partie distale ou proximale du corps 12. Dans le cas de la configuration proximale (comme sur la figure 1), l'acheminement de la lumière de la zone à examiner jusqu'à la caméra optique pourra être réalisé par un réseau de fibres optiques ou tout autre dispositif optique. La caméra optique, composée de plusieurs photodiodes est configurée pour convertir les ondes lumineuses capturées en signaux électriques exploitables par un module de traitement (voir ci-après).

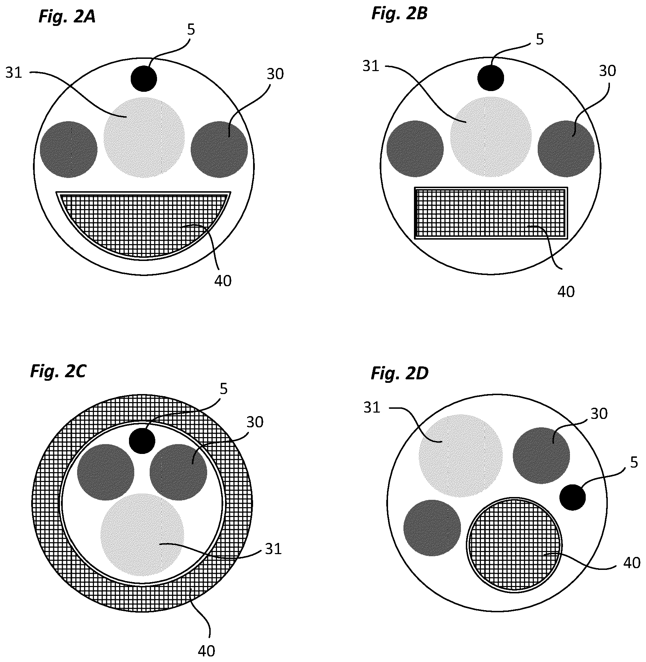

Les figures 2A à 2D montrent plusieurs exemples de mise en œuvre et de positionnement des composants du dispositif optique et du dispositif ultrasonore. L'observation de la zone à examiner est par exemple réalisée au travers d'un récepteur optique 31, pouvant intégrer une ou plusieurs lentilles.

Le dispositif ultrasonore 4 comporte plusieurs transducteurs ultrasonores 40, avantageusement une matrice de transducteurs ultrasonores. Ces transducteurs ultrasonores 40 peuvent être des corps piézo-électriques ou des transducteurs ultrasonores micro-usinés capacitifs (appelés CMUT). Le matériau du corps piézo-électrique peut être en quartz, en céramique (titanate-zirconate de plomb ou polyfluorure de vinylidène) ou en polymère. La matrice de transducteurs ultrasonores 40 peut prendre différentes formes, cette forme pouvant être adaptée à celle de la section transversale du corps de la sonde bimodale.

A titre d'exemple, la matrice de transducteurs peut présenter une forme semi-circulaire (figure 2A), rectangulaire (figure 2B), annulaire (figure 2C) ou circulaire (figure 2D).

Le dispositif ultrasonore 4 est contrôlé pour insonifier une région d'intérêt avec des ondes ultrasonores : dans un mode élastographique, une onde de cisaillement plane est induite dans la région d'intérêt par l'insonification, et la propagation du « plan » de cisaillement est mesuré en temps réel à haute vitesse par imagerie. La partie ultrasonore de la sonde 1 fonctionne ainsi en émission-réception.

Selon l'invention, la sonde 1 bimodale comporte une partie 13 montée mobile en coulissement par rapport au corps 12 de la sonde suivant l'axe longitudinal (X) de la sonde.

Cette partie mobile 13 comporte une extrémité distale, située du côté de la partie distale 11 de la sonde. A cette extrémité distale, la partie mobile 13 porte le dispositif ultrasonore 4. Il est ainsi possible de venir régler la position du dispositif ultrasonore 4 par rapport au reste du corps 12 de la sonde.

Du côté proximal, la partie mobile 13 peut comporter un manche de préhension 132, pouvant être utilisé par l'opérateur pour faire coulisser la partie mobile 13 le long de son axe.

Cette partie mobile 13 permet de déplacer le dispositif ultrasonore 4 et d'approcher la matrice de transducteurs ultrasonores 40 au plus près des tissus à examiner pour permettre l'imagerie ultrasonore, alors que le faisceau lumineux 30 du dispositif optique peut être émis d'une position plus éloignée afin de conserver un champ d'observation adapté à la chirurgie. Il est ainsi possible de jouer sur la position de chacun des deux dispositifs, en n'utilisant qu'une seule sonde 1.

Selon l'invention, la sonde 1 peut comporter des moyens de réglage (non représenté) de la position longitudinale de la partie mobile par rapport au corps de la sonde. Ces moyens de réglage peuvent être formés de plusieurs crans ou de toute autre solution équivalente. Un organe de blocage 131 en position peut être intégré à la sonde. Sur la figure 1, on peut voir que la partie mobile est déplacée longitudinalement d'une distance D_x.

Un capteur de déplacement 130 peut également être intégré à la sonde 1 pour détecter la position de la partie mobile 13 par rapport au reste du corps 12 de la sonde.

Le coulissement de la partie mobile 13 de la sonde 1 pourra se faire de manière manuelle ou motorisée. La sonde 1 peut ainsi intégrer un moteur électrique, contrôlé pour permettre un déplacement en translation de la partie mobile 13. Le transfert du mouvement du moteur électrique vers la partie mobile 13 peut être mis en œuvre à l'aide d'un mécanisme de crémaillère et/ou vis sans fin ou de toute autre solution équivalente.

De manière avantageuse, la sonde 1 peut comporter un canal, dit canal opérateur 5 (figures 2A à 2D), qui s'étend à travers son corps 12, suivant la direction longitudinale (X). Ce canal 5 peut notamment servir à l'opérateur soit pour injecter des substances servant au nettoyage ou pour toute autre opération, soit pour aspirer les déchets liquidiens ou solides et permet ainsi à l'opérateur d'atteindre les tissus à examiner depuis l'extérieur de la sonde 1.

La partie mobile 13 peut être munie d'au moins un capteur de pression (non représenté) permettant de mesurer la force appliquée sur les tissus au moment de la mesure élastographique. Le capteur de pression a pour objectif de ne pas induire de lésion des tissus par contact de la sonde 1, d'obtenir une déformation minimale des tissus et avantageusement de pouvoir toujours appliquer une force de même intensité lors de chaque mesure ultrasonore.

La sonde peut disposer d'une batterie électrique logée dans son corps 12, pour alimenter ses différents composants (dispositif optique, dispositif ultrasonore) et/ou être raccordée à une source d'alimentation externe.

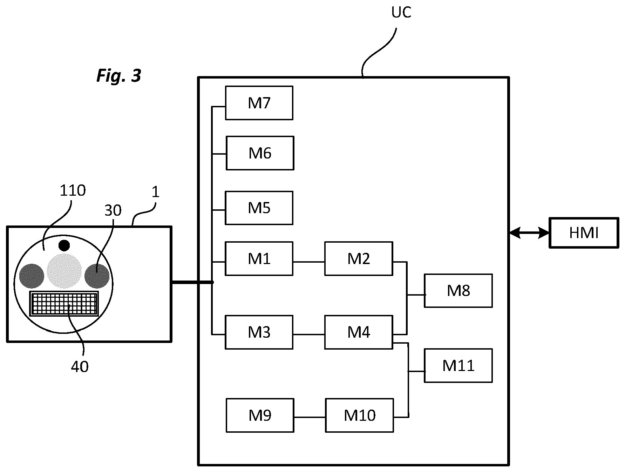

En référence à la figure 3, pour fonctionner, la sonde 1 bimodale est intégrée à un système de diagnostic plus global.

De manière non limitative, en plus de la sonde 1 bimodale, le système de diagnostic peut comporter au moins une unité de contrôle et de traitement UC et une interface homme-machine HMI.

L'interface homme-machine HMI permet notamment de saisir des données physiologiques relatives au patient suivi, de configurer le fonctionnement de la sonde 1 (par exemple en réglant la position de la partie mobile 13 portant le dispositif ultrasonore 4) et le type de traitement appliqué aux données ultrasonores (mode B, élastographie, Doppler).

La sonde 1 est par exemple reliée de manière amovible à ladite unité de contrôle et de traitement UC.

L'unité de contrôle et de traitement UC comporte :
- Un module M1 d'émission/réception de données auquel est connecté le dispositif optique de la sonde ; ce module M1 est chargé de contrôler la source lumineuse (par exemple une ou plusieurs diodes électroluminescentes) pour émettre le faisceau lumineux 30 en direction des tissus à examiner et de recevoir les données représentatives des flux lumineux reçus par la caméra optique intégré au dispositif optique 3 pour les convertir en données électriques ; chaque diode électroluminescente peut par exemple être commandée de manière individualisée ;
- Un module M2 de traitement des données représentatives du flux lumineux capté par le dispositif optique ;
- Un module M3 d'émission/réception de données auquel est connecté le dispositif ultrasonore de la sonde ; ce module est chargé de contrôler plusieurs transducteurs ultrasonores 40 pour émettre des ultrasons en direction des tissus à examiner et de recevoir les données représentatives des ondes ultrasonores reçues par les transducteurs ultrasonores 40 ;
- Au moins un module M4 de traitement des données représentatives des ondes ultrasonores captées par les transducteurs ultrasonores, ce module M4 de traitement pouvant être configuré pour effectuer un traitement mode B classique, un traitement par élastographie et/ou de type Doppler (optionnel) ;
- Un module M5 de mesure de la position de la partie mobile 13 portant le dispositif ultrasonore par rapport au corps 12 ; chargé d'envoyer ou non des données de position au module M6.
- Un module M6 de contrôle de la position de la partie mobile 13, chargé de déplacer cette dernière.
- Un module M7 de pilotage du bras articulé 2.
- Un module M8 de superposition des images obtenues via le mode optique et via le mode ultrasonore ; ce module pourra notamment s'appuyer sur les différents modes de traitement ultrasonores possibles mentionnés ci-dessus (mode B, élastographie, Doppler) ;
- De manière optionnelle, un module M9 d'acquisition d'images obtenues par IRM et un module M10 de traitement de ces images ;
- De manière optionnelle, un module M11 de superposition des images obtenues par IRM et de celles obtenues par un mode de traitement ultrasonore ;

Le mode B correspond au mode de visualisation classique employé lors d'une échographie.

Le module M4 de traitement des données représentatives des ondes ultrasonores permet de générer une cartographie de rigidité des tissus. Ces images sont par exemple des échographies en trois dimensions en regard de la région d'intérêt.

Cette cartographie peut refléter des mesures brutes ou des mesures relatives, par zones. L'interface homme-machine HMI peut par exemple être configurée pour régler un ou plusieurs seuils de rigidité des tissus et de configurer les images obtenues en tenant compte de chaque seuil fixé. Par exemple, il est possible d'afficher les zones observées avec des couleurs différentes en tenant compte de leur niveau de rigidité par rapport à chacun des seuils pré-enregistrés. Il sera ainsi plus facile de discriminer les zones pathologiques des zones saines.

Il faut noter que le module M8 de superposition des images pourra adapter les images générées grâce au dispositif ultrasonore en tenant compte de sa position déterminée par le capteur de déplacement.

Chaque module M8, M11 de superposition des images permet de superposer des images obtenues à l'aide du dispositif optique 3 et du dispositif ultrasonore 4 intégrés à la même sonde 1. Autrement dit, la sonde de l'invention permet de recueillir en temps réel et de manière simultanée les images via ces deux canaux d'imagerie. Il sera également possible de venir superposer des images récupérées via IRM.

De manière non limitative, la sonde 1 peut ainsi être employée pour localiser la lésion puis pour confirmer le diagnostic par :
- Superposition de l'image élastographique et de l'image en mode B (mode B=mode de visualisation de l'échographie) ;
- Superposition de l'image obtenue via les données optiques et de l'image élastographique ;
- Superposition de l'image obtenue par IRM et de l'image élastographique ;
- Spatialisation et calcul du volume de la lésion : combinaison des différentes modalités et étude de corrélation (zones communes et spécifiques à chaque mode).

Ladite sonde peut également aider dans les situations suivantes :
- Au guidage de l'exérèse par projection de la lésion sur l'image en 2D obtenue grâce aux données optiques ;
- Au suivi de la résection de la lésion grâce à la spatialisation et le calcul du volume de la lésion ;
- Au contrôle final de la résection ;

Les applications médicales visées par la présente invention sont les adénomes hypophysaires, les tumeurs cérébrales au sens large (méningiomes, gliomes, cavernomes, craniopharyngiomes, etc.), les tumeurs au sens large (foie, sein, poumon, etc.) et notamment les récidives des tumeurs citées précédemment. Dans l'exemple des tumeurs de l'hypophyse et plus particulièrement du microadénome hypophysaire à ACTH, le système de diagnostic permet dans un premier temps de détecter la lésion par une mesure de la rigidité des tissus balayés, dans un second temps de guider en temps réel l'exérèse en repérant grâce à la fonction Doppler du dispositif les structures vasculaires importantes, et enfin de permettre une exérèse complète de l'adénome en délimitant précisément les contours de la lésion dans le but de la retirer en totalité sans retirer de tissus sains et en évitant ainsi une exploration chirurgicale délétère pour la glande.

L'invention présente de nombreux avantages parmi lesquels :
- On obtient une sonde ayant une architecture compacte tout en disposant de la bimodalité optique et ultrasonore ;
- On obtient une sonde disposant d'une solution permettant d'aller au contact des tissus à examiner pour réaliser l'imagerie ultrasonore puis de revenir à sa position initiale pour laisser libre le champ de vision de la modalité optique.

## Revendications

1. Système de diagnostic capable de générer des images à partir de données ultrasonores et de données optiques, ledit système comprenant une sonde (1) de diagnostic bimodale et une unité de contrôle et de traitement (UC) à laquelle est connectée ladite sonde de diagnostic bimodale étant employée pour examiner des tissus biologiques et comprenant :
- Un corps (12) présentant une forme allongée suivant un axe longitudinal (X),
- Le corps (12) comportant une partie distale (11) destinée à venir au plus près des tissus à examiner,
- Au moins un dispositif ultrasonore (4) comportant une matrice de transducteurs (40) ultrasonores, ladite matrice de transducteurs ultrasonores (40) étant commandée pour émettre des signaux ultrasonores en direction des tissus à examiner et pour convertir les signaux ultrasonores réfléchis en signaux électriques,
- Au moins un dispositif optique (3) comportant une source lumineuse, ladite au moins une source lumineuse étant contrôlée pour émettre un faisceau lumineux (30) en direction des tissus à examiner, le dispositif optique comprenant également au moins un capteur chargé de capter les signaux lumineux diffusés par les tissus,
- Le dispositif optique (3) étant agencé pour émettre ledit faisceau lumineux (30) au niveau de la partie distale (11) de la sonde,
- La sonde (1) comportant une partie (13) montée mobile en coulissement dans ledit corps (12) suivant une direction parallèle à l'axe longitudinal (X), ladite partie mobile (13) comprenant une extrémité distale portant le dispositif ultrasonore (4),
- La sonde (1) comportant des moyens de réglage de la position longitudinale de ladite partie mobile (13),
- **Caractérisé en ce que** :
- Le système comporte des moyens d'acquisition de la position longitudinale de la partie mobile (13) de la sonde et **en ce que** l'unité de contrôle et de traitement (UC) est configurée pour déterminer la position longitudinale de ladite matrice de transducteurs ultrasonores (40) par rapport au dispositif optique (3)
- L'unité de contrôle et de traitement (UC) est configurée pour corriger l'image obtenue à l'aide du dispositif ultrasonore (4) et/ou l'image obtenue à l'aide du dispositif optique (3) en tenant compte de la position longitudinale de ladite matrice de transducteurs ultrasonores (40) par rapport au dispositif optique (3).

2. Système selon la revendication 1, **caractérisée en ce que** la sonde comporte un capteur de position agencé pour détecter la position longitudinale de la partie mobile (13).

3. Système selon la revendication 1 ou 2, **caractérisée en ce que** la sonde comporte un dispositif de blocage (131) en coulissement de la partie mobile (13).

4. Système selon l'une des revendications 1 à 3, **caractérisé en ce que** la sonde comporte au moins un capteur de pression mécanique situé à l'extrémité distale de la partie mobile (13).

5. Système selon l'une des revendications 1 à 4, **caractérisée en ce que** le dispositif ultrasonore (4) comporte une matrice de transducteurs ultra-sonores (40).

6. Système selon l'une des revendications 1 à 5, **caractérisée en ce que** la sonde comporte un canal dit canal opérateur (5) intégré audit corps (12) de la sonde et s'étendant selon une direction parallèle à l'axe longitudinal (X).

7. Système selon l'une des revendications 1 à 6, **caractérisé en ce que** l'unité de contrôle et de traitement (UC) comporte un module (M8) de superposition des images obtenues grâce au dispositif optique (3) et des images obtenues grâce au dispositif ultrasonore (4).

8. Système selon l'une des revendications 1 à 7, **caractérisé en ce que** l'unité de contrôle et de traitement (UC) comporte un module (M11) de superposition des images obtenues grâce au dispositif ultrasonore (4) et des images obtenues par IRM.

9. Système selon l'une des revendications 1 à 8, **caractérisé en ce qu'**il comporte un module (M4) de traitement des images obtenues grâce au dispositif ultrasonore, configuré pour distinguer les différentes rigidités de tissus examinés.

## Patentansprüche

1. Diagnosesystem, das in der Lage ist, Bilder ausgehend von Ultraschalldaten und von optischen Daten zu erzeugen, wobei das System eine bimodale Diagnosesonde (1) umfasst und eine Steuerungs- und Verarbeitungseinheit (UC), an welche die bimodale Diagnosesonde angeschlossen ist, die dazu verwendet wird, biologische Gewebe zu untersuchen und umfasst:
- Einen Körper (12), der eine entlang einer Längsachse (X) langgestreckte Form aufweist,
- Wobei der Körper (12) einen distalen Teil (11) beinhaltet, der dazu bestimmt ist, so nah wie möglich an die zu untersuchenden Gewebe zu gelangen,
- Mindestens eine Ultraschallvorrichtung (4), de eine Ultraschallwandlermatrix (40) beinhaltet, wobei die Ultraschallwandlermatrix (40) so gesteuert wird, dass sie Ultraschallsignale in Richtung der zu untersuchenden Gewebe aussendet und die reflektierten Ultraschallsignale in elektrische Signale umwandelt,
- Mindestens eine optische Vorrichtung (3), die eine Lichtquelle beinhaltet, wobei die mindestens eine Lichtquelle so gesteuert wird, dass sie einen Lichtstrahl (30) in Richtung der zu untersuchenden Gewebe aussendet, wobei die optische Vorrichtung auch mindestens einen Sensor umfasst, der die Aufgabe hat, die von den Geweben gestreuten Lichtsignale zu erfassen;
- Wobei die optische Vorrichtung (3) so eingerichtet ist, dass sie den Lichtstrahl (30) an dem distalen Teil (11) der Sonde aussendet,
- Wobei die Sonde (1) einen Teil (13) beinhaltet, der in dem Körper (12) entlang einer zu der Längsachse (X) parallelen Richtung verschieblich gelagert ist, wobei der bewegliche Teil (13) ein distales Ende umfasst, das die Ultraschallvorrichtung (4) trägt,
- Wobei die Sonde (1) Mittel zum Einstellen der Längsposition des beweglichen Teils (13) beinhaltet,
- **Dadurch gekennzeichnet, dass**:
- Das System Mittel zum Erfassen der Längsposition des beweglichen Teils (13) der Sonde beinhaltet und dass die Steuerungs- und Verarbeitungseinheit (UC) dazu ausgestaltet ist, die Längsposition der Ultraschallwandlermatrix (40) in Bezug auf die optische Vorrichtung (3) zu bestimmen.
- Die Steuerungs- und Verarbeitungseinheit (UC) dazu ausgestaltet ist, das mithilfe der Ultraschallvorrichtung (4) erhaltene Bild und/oder das mithilfe der optischen Vorrichtung (3) erhaltene Bild unter Berücksichtigung der Längsposition der Ultraschallwandlermatrix (40) in Bezug auf die optische Vorrichtung (3) zu korrigieren.

2. System nach Anspruch 1, **dadurch gekennzeichnet, dass** die Sonde einen Positionssensor beinhaltet, der dazu eingerichtet ist, die Längsposition des beweglichen Teils (13) zu detektieren.

3. System nach Anspruch 1 oder 2, **dadurch gekennzeichnet, dass** die Sonde eine Verschiebesicherungsvorrichtung (131) für den beweglichen Teil (13) beinhaltet.

4. System nach einem der Ansprüche 1 bis 3, **dadurch gekennzeichnet, dass** die Sonde mindestens einen mechanischen Drucksensor beinhaltet, der an dem distalen Ende des beweglichen Teils (13) gelegen ist.

5. System nach einem der Ansprüche 1 bis 4, **dadurch gekennzeichnet, dass** die Ultraschallvorrichtung (4) eine Ultraschallwandlermatrix (40) beinhaltet.

6. System nach einem der Ansprüche 1 bis 5, **dadurch gekennzeichnet, dass** die Sonde einen als Operationskanal (5) bezeichneten Kanal aufweist, der in den Körper (12) der Sonde integriert ist und sich entlang einer zur Längsachse (X) parallelen Richtung erstreckt.

7. System nach einem der Ansprüche 1 bis 6, **dadurch gekennzeichnet, dass** die Steuerungs- und Verarbeitungseinheit (UC) ein Modul (M8) zur Überlagerung der mit der optischen Vorrichtung (3) erhaltenen Bilder und der mit der Ultraschallvorrichtung (4) erhaltenen Bilder beinhaltet.

8. System nach einem der Ansprüche 1 bis 7, **dadurch gekennzeichnet, dass** die Steuerungs- und Verarbeitungseinheit (UC) ein Modul (M11) zur Überlagerung der mit der Ultraschallvorrichtung (4) erhaltenen Bilder und der durch MRT erhaltenen Bilder beinhaltet.

9. System nach einem der Ansprüche 1 bis 8, **dadurch gekennzeichnet, dass** es ein Modul (M4) zur Verarbeitung der mit der Ultraschallvorrichtung erhaltenen Bilder beinhaltet, das dazu ausgestaltet ist, die verschiedenen Steifigkeiten von untersuchten Geweben zu unterscheiden.

## Claims

1. Diagnostic system capable of generating images from ultrasonic data and optical data, said system comprising a bimodal diagnostic probe (1) and a control and processing unit (UC) to which said bimodal diagnostic probe is connected being used to examine biological tissues and comprising:
- a body (12) having an elongate shape along a longitudinal axis (X),
- the body (12) comprising a distal part (11) intended to come as close as possible to the tissues to be examined,
- at least one ultrasonic device (4) comprising a matrix array of ultrasonic transducers (40), said matrix array of ultrasonic transducers (40) being controlled to emit ultrasonic signals towards the tissues to be examined and to convert the reflected ultrasonic signals into electrical signals,
- at least one optical device (3) comprising a light source, said at least one light source being controlled to emit a light beam (30) towards the tissues to be examined, the optical device also comprising at least one sensor responsible for capturing the light signals scattered by the tissues,
- the optical device (3) being arranged to emit said light beam (30) at the distal part (11) of the probe,
- the probe (1) comprising a part (13) slidably mounted in said body (12) in a direction parallel to the longitudinal axis (X), said movable part (13) comprising a distal end bearing the ultrasonic device (4),
- the probe (1) comprising means for adjusting the longitudinal position of said movable part (13),
- **characterized in that**:
- the system comprises means for acquiring the longitudinal position of the movable part (13) of the probe, and **in that** the control and processing unit (UC) is configured to determine the longitudinal position of said matrix array of ultrasonic transducers (40) with respect to the optical device (3),
- the control and processing unit (UC) is configured to correct the image obtained with the aid of the ultrasonic device (4) and/or the image obtained with the aid of the optical device (3) taking account of the longitudinal position of said matrix array of ultrasonic transducers (40) with respect to the optical device (3).

2. System according to Claim 1, **characterized in that** the probe comprises a position sensor arranged to detect the longitudinal position of the movable part (13).

3. System according to Claim 1 or 2, **characterized in that** the probe comprises a device (131) for slidingly blocking the movable part (13).

4. System according to any of Claims 1 to 3, **characterized in that** the probe comprises at least one mechanical pressure sensor located at the distal end of the movable part (13).

5. System according to any of Claims 1 to 4, **characterized in that** the ultrasonic device (4) comprises a matrix array of ultrasonic transducers (40).

6. System according to any of Claims 1 to 5, **characterized in that** the probe comprises a channel, referred to as operating channel (5), integrated into said body (12) of the probe and extending in a direction parallel to the longitudinal axis (X).

7. System according to any of Claims 1 to 6, **characterized in that** the control and processing unit (UC) comprises a module (M8) for superposing images obtained by means of the optical device (3) and images obtained by means of the ultrasonic device (4).

8. System according to any of Claims 1 to 7, **characterized in that** the control and processing unit (UC) comprises a module (M11) for superposing images obtained by means of the optical device (4) and images obtained by MRI.

9. System according to any of Claims 1 to 8, **characterized in that** it comprises a module (M4) for processing images obtained by means of the ultrasonic device, configured to distinguish the different rigidities of tissues examined.
